(19) Europäisches Patentamt
European Patent Office
Office européen des brevets

(11) Veröffentlichungsnummer: **0 056 860**
**B1**

(12) # EUROPÄISCHE PATENTSCHRIFT

(45) Veröffentlichungstag der Patentschrift: **06.03.85**

(51) Int. Cl.[4]: **C 07 D 249/08,** A 01 N 43/64, A 01 N 43/50

(21) Anmeldenummer: **81110351.4**

(22) Anmeldetag: **11.12.81**

(54) **Substituierte Azolyl-glykolsulfonate, diese enthaltende Fungizide und Verfahren zu ihrer Herstellung.**

(30) Priorität: **08.01.81 DE 3100260**

(43) Veröffentlichungstag der Anmeldung: **04.08.82 Patentblatt 82/31**

(45) Bekanntmachung des Hinweises auf die Patenterteilung: **06.03.85 Patentblatt 85/10**

(84) Benannte Vertragsstaaten: **AT BE CH DE FR GB IT LI LU NL SE**

(56) Entgegenhaltungen:
**EP - A - 0 000 752**
**EP - A - 0 001 414**
**EP - A - 0 003 049**
**EP - A - 0 004 303**
**EP - A - 0 021 345**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Reuther, Wolfgang, Dr., Am Pferchelhang 16, D-6900 Heidelberg (DE)**
Erfinder: **Ruland, Alfred, Dr., Schriesheimer Strasse 11, D-6945 Hirschberg (DE)**
Erfinder: **Pommer, Ernst-Heinrich, Dr., Berliner Platz 7, D-6703 Limburgerhof (DE)**

**EP 0 056 860 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Triazolyl-glykolsulfonate, Verfahren zu ihrer Herstellung und ihre Verwendung als Fungizide.

Es ist bekannt, daß Imidazolylether, z. B. das 1-(2-(2,4-Dichlorphenyl)-2-(2-propenyloxy)-ethyl)-1H-imidazol (GB-PS 1 318 590), eine fungizide Wirksamkeit haben. Die Wirkung dieser Stoffe ist jedoch unbefriedigend. Es ist ferner bekannt, das Zink-ethylen-bisdithiocarbamat als Fungizid zu verwenden (Chemical Week, Heft vom 26. Juli 1972, Seite 41). Seine Wirkung ist jedoch unbefriedigend. Imidazolylether-Verbindungen mit fungizider Wirkung sind aus EP-A-1 414, EP-A-3 049 und EP-A-4 303 bekannt.

Es wurde nun gefunden, daß Triazolyl-glykolsulfonate der Formel 1

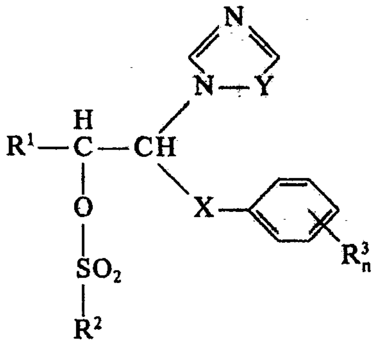

worin

$R^1$ Alkyl mit 1—6 C-Atomen, gegebenenfalls substituiert durch Fluor, Chlkor, Brom, Jod oder $R^1$ Alkoxy mit 1—4 C-Atomen oder Phenyl bedeutet, wobei der Phenylrest durch Fluor, Chlor, Brom, Jod, Cyan, Alkyl mit 1—6 C-Atomen, Alkoxy mit 1—4 C-Atomen, Phenyl oder Phenoxy substituiert sein kann,

$R^2$ die gleichen Bedeutungen wie $R^1$ hat, wobei $R^1$ und $R^2$ im Einzelfall gleich oder verschieden sind,

$R^3$ Wasserstoff, Halogen, Alkyl mit 1—4 C-Atomen, Alkoxy mit 1—4 C-Atomen, Cyan, Phenyl oder Phenoxy bedeutet, wobei der Phenyl- oder der Phenoxyrest selbst wieder durch Halogen oder Alkyl mit 1—4 C-Atomen substituiert sein kann,

n eine ganze Zahl von 1 bis 5, X S oder O und Y N oder CH bedeutet,

eine gute fungizide Wirkung haben.

Die Verbindungen der Formel 1 besitzen zwei (evtl. auch mehr) Chiralitätszentren. Sie fallen im Rahmen der Synthese als Diastereomerengemische an, sofern die Ausgangsalkohole ebenfalls als Diastereomerengemische eingesetzt werden. Durch fraktionierte Kristallisation lassen sich reine Diastereomere erhalten, die ebenfalls von der Erfindung miterfaßt werden.

Die erfindungsgemäßen Verbindungen lassen sich aus den Alkoholen der Formel

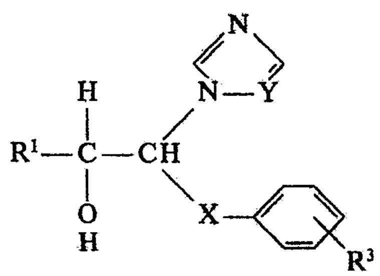

herstellen, die nach bekannten Verfahren hergestellt werden können und in der Literatur beschrieben sind und die durch Umsetzung mit Alkalihydriden oder Amiden oder mit Basen wie NaOH, KOH in Gegenwart von Kattalysatoren wie quartären Ammonium- oder Phosphoniumsalzen in aprotischen Lösungsmitteln, z. B. THF (Tetrahydrofuran), DMF (Dimethylformamid), Dioxan, zu den entsprechenden Alkoholaten der Formel

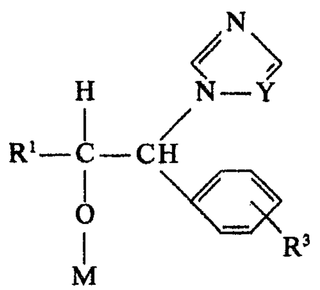

M ist Natrium oder Kalium

umgesetzt werden können, und diese werden mit Sulfonsäurehalogeniden der Formel

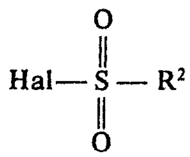

in der Hal ein Halogenatom bedeutet,

vorzugsweise mit Sulfonsäurechloriden, in einem der genannten Lösungsmittel zu den Verbindungen der Formel 1 umgesetzt.

Beispiel 1

1-(4-Chlorphenoxy)-3,3-dimethyl-2-(4-methylphenylsulfonyl)-
1-(1,2,4-triazol-1-yl)-butan

44,6 g (0,15 Mol) 1-(1,2,4-Triazol-1-yl)-1-(4-chlorphenoxy)-3,3-dimethyl-butan-3-ol als Diastereomerengemisch werden in 500 ml absolutem THF (Tetrahydrofuran) gelöst und unter Rühren bei Raumtemperatur mit 4,5 g Natriumhydrid (~80%ig) versetzt. Danach wird 6—8 Stunden bei 40—50°C gerührt und nach dem Abkühlen 28,5 g (0,15 Mol) p-Toluolsulfochlorid, gelöst in möglichst wenig THF, zugetropft. Man rührt weitere 5—6 Stunden bei Raumtemperatur, hydrolysiert dann mit Wasser, extrahiert mehrfach mit 500 ml Methylenchlorid, trocknet die organische Phase über Natriumsulfat und destilliert das Lösungsmitel im Vakuum ab. Der verbleibende Rückstand wird aus Essigester fraktioniert kristallisiert, wobei 23,5 g (35% d. Th.) an Diastereoisomerengemisch erhalten werden. Schmp.: 156—157°C (Verbindung Nr. 1).

Entsprechend Beispiel 1 wurden folgende weitere Derivate hergestellt.

| Verbindung Nr. | $R^1$ | $R^2$ | $R^3$ | n | Y | X | Schmp. in °C |
|---|---|---|---|---|---|---|---|
| 2 | $(CH_3)_3C-$ | 4-Methyl-phenyl | 2,4,5-Cl | 3 | N | O | 122—126 |
| 3 | $(CH_3)_3C-$ | desgl. | 4-Br | 1 | N | O | 168—170 |
| 4 | $(CH_3)_3C-$ | desgl. | 2-Cl | 1 | N | O | 82—85 |
| 5 | $(CH_3)_3C-$ | desgl. | 3,5-Cl | 2 | N | O | 210—212 |
| 6 | $(CH_3)_3C-$ | desgl. | 4-$C(CH_3)_3$ | 1 | N | O | 113 |
| 7 | $(CH_3)_3C-$ | desgl. | 3-$CF_3$ | 1 | N | O | 136—137 |
| 8 | $(CH_3)_3C-$ | desgl. | 4-Phenyl | 1 | N | O | 155—156 |
| 9 | $(CH_3)_3C-$ | desgl. | 2,4-Cl | 2 | N | O | 134—136 |
| 10 | Phenyl | desgl. | 4-Br | 1 | N | O | 86—89 |
| 11 | Phenyl | desgl. | 2-Cl | 1 | N | O | 126:129 |
| 12 | Phenyl | desgl. | 4-Phenyl | 1 | N | O | 84—87 |
| 13 | $(CH_3)_3C-$ | desgl. | 4-Br | 1 | N | S | 139—141*) |
| 14 | $(CH_3)_3C-$ | desgl. | 4-Br | 1 | N | S | 127—129*) |

*) Diastereoisomerenpaar

Die Wirkstoffe eignen sich besonders zur Bekämpfung von Pilzerkrankungen an verschiedenen Kulturpflanzen, z. B. Erysiphe graminis an Getreide, Uncinula necator an Reben, Podosphaera leucotricha an Äpfeln, Erysiphe cichoracearum an Kürbisgewächsen, Mycosphaerella musicola an Bananen, Phytophthora infestans an Tomaten und Kartoffeln, Plasmopara viticola an Reben, Pseudoperonospora humuli an Hopfen.

Einige der erfindungsgemäßen Wirkstoffe sind systemisch wirksam; die zur Bekämpfung phytopathogener Pilze erforderlichen Aufwandmengen liegen zwischen 0,025 und 2 kg Wirkstoff/ha Kulturfläche.

Die neuen Wirkstoffe eignen sich auch zum Schutz von Holz gegen den Befall durch holzzerstörende Pilze, wie Coniophora puteana, Lenzites trabea, Trametes versicolor. Die Aufwandmengen betragen je nach Art des gewünschten Effektes 0,001 bis 5% (Gewichtsprozent), bezogen auf das Gewicht des zu schützenden Materials, vorzugsweise jedoch zwischen 0,01 und 3%.

Die Anwendung als Fungizid erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Streichen, Tränken, Vernebeln, Verstäuben, Verstreuen oder Gießen. Die Anwendungsformen richten sich ganz nach den Verwendungszwecken; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solze oder in einem Öl oder Lösungsmittel gelöst, mittels Netz-, Haft-, Dispergier- oder Emulgiermittel in Wasser homogenisiert werden.

Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Fungizide enthalten z. B. 5 bis 95% (Gew.-%) insbesondere 10 bis 80% Wirkstoff.

An oberflächenaktiven Stoffen sind zu nennen:

Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol-, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreibemehle, Baumrinden-, Holz- und Nußschalenmehl, Cellulosepulver und andere feste Trägerstoffe.

## Beispiel a

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

## Beispiel b

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

## Beispiel c

20 Gewichtsteile der Verbindung 3 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

## Beispiel d

20 Gewichtsteile der Verbindung 1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht.

## Beispiel e

80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel f

5 Gewichtsteile der Verbindung 1 werden mit 95 Gewichtsteilen feinteiligem Kaolin innig vermischt. Man erhält auf diese Weise ein Stäubemittel, das 5 Gewichtsprozent des Wirkstoffs enthält.

Beispiel g

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel h

40 Gewichtsteile des Wirkstoffs 1 werden mit 10 Teilen Natriumsalz eines Phensolsulfonsäure-harnstoff-formaldehyd-kondensats, 2 Teilen Kieselgel und 48 Teilen Wasser innig vermischt. Man erhält eine stabile wäßrig Dispersion. Durch Verdünnen mit 100 000 Gewichtsteilen Wasser erhält man eine wäßrige Dispersion, die 0,04 Gewichtsprozent Wirkstoff enthält.

Beispiel i

20 Teile des Wirkstoffs 1 werden mit 12 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die Fungizide können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Dithiocarbamate und deren Derivate, wie
- Ferridimethyldithiocarbamat,
- Zinkdimethyldithiocarbamat,
- Manganethylenbisdithiocarbamat,
- Mangan-Zink-ethylendiamin-bis-dithiocarbamat,
- Zinkethylenbisdithiocarbamat,
- Tetramethylthiuramdisulfide,
- Ammoniak-Komplex von Zink-(N,N-ethylen-bis-dithiocarbamat) und N,N'-Polyethylen-bis-(thiocarbamoyl)-disulfid,
- Zink-(N,N'-propylen-bis-dithiocarbamat),
- Ammoniak-Komplex von Zink-(N,N'-propylen-bis-dithiocarbamat) und N,N'-Polypropylen-bis-(thiocarbamoyl)-disulfid;

Nitroderivate, wie
- Dinitro-(1-methylheptyl)-phenylcrotonat,
- 2-sec-Butyl-4,6-dinitrophenyl-3,3-dimethylacrylat,
- 2-sec-Butyl-4,6-dinitrophenyl-isopropylcarbonat;

heterocyclische Strukturen, wie
- N-Trichlormethylthio-tetrahydrophthalimid,
- N-Trichlormethylthio-phthalimid,
- 2-Heptadecyl-2-imidazolin-acetat,
- 2,4-Dichlor-6-(o-chloranilino)-s-triazin,
- O,O-Diethyl-phthalimidophosphonothioat,
- 5-Amino-1-(bis-dimethylamino)-phosphinyl)-3-phenyl-1,2,4-triazol,
- 5-Ethoxy-3-trichlormethyl-1,2,4-thiadiazol,
- 2,3-Dicyano-1,4-dithioanthrachinon,
- 2-Thio-1,3-dithio-(4,5-b)-chinoxalin,
- 1-(Butylcarbamoyl)-2-benzimidazol-carbaminsäuremethylester,
- 2-Methoxycarbonylamino-benzimidazol,

2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie

Dodecylguanidinacetat,
3-(3-(3,5-Dimethyl-2-oxycyclohexyl)-2-hydroxyähtyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyetyl)-N'-imidazolyl-harnstoff.

Die folgenden Versuche erläutern die gute fungizide Wirkung der Wirkstoffe im Vergleich zu bekannten Wirkstoffen.

Als Vergleichsmittel wurden folgende bekannte Verbindungen verwendet:

| | |
|---|---|
| 1-(2'-(2'',4''-Dichlorphenyl)-2'-(2''-propylenoxy)-ethyl-1H-imidazol | (Verbindung A) |
| Zink-etylen-bis-dithiocarbamat | (Verbindung B) |

### Versuch 1

#### Wirksamkeit gegen Weizenmehltau

Blätter von in Töpfen gewachsenen Weizenkeimlingen der Sorte »Jubilar« werden mit wäßrigen Emulsionen aus 80% (Gewichtsprozent) Wirkstoff und 20% Emulgiermittel (bezogen auf die Trockensubstanz) besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltauentwicklung ermittelt.

Bei diesem Versuch zeigten die neuen Verbindungen 1 und 6 eine bessere fungizide Wirkung als der bekannte Wirkstoff A.

### Versuch 2

#### Wirksamkeit gegen Gurkenmehltau

Blätter von in Töpfen gewachsenen Gurkenkeimlingen werden mit wäßrigen Emulsionen aus 80% Wirkstoff und 20% Emulgiermittel (bezogen auf die Trockensubstanz) besprüht und nach dem Antrocknen des Spritzbelages mit Oidien (Sporen) des Gurkenmehltaus (Erysiphe cichoracearum) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

Bei diesem Versuch zeigten die Wirkstoffe 1, 3 und 4 eine sehr gute fungizide Wirkung.

Versuch 3

Wirksamkeit gegen Phytophthora infestans an Tomaten

Blätter von Tomatenpflanzen der Sorte »Professor Rudloff« werden mit wäßrigen Suspensionen, die 80% (Gewichtsprozent) des zu prüfenden Wirkstoffes und 20% Natriumligninsulfonat in der Trockensubstanz enthalten, besprüht. Es werden 0,025-, 0,006- und 0,0015%ige Spritzbrühen (berechnet auf die Trockensubstanz) verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann:

Bei diesem Versuch zeigte der neue Wirkstoff 6 eine bessere fungizide Wirkung als der bekannte Wirkstoff B.

**Patentansprüche für die Vertragsstaaten: BE, CH, DE, FR, GB, IT, LI, LU, NL, SE**

1. Azolyl-glykolsulfonat der allgemeinen Formel

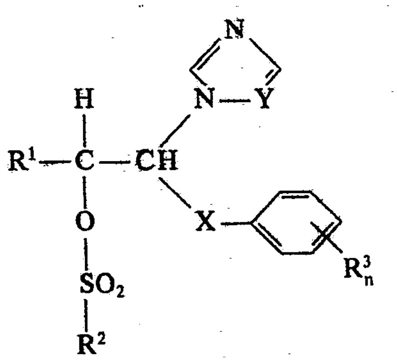

in der

$R^1$ Alkyl mit 1–6 C-Atomen, gegebenenfalls substituiert durch Fluor, Chlor, Brom, Jod oder $R^1$ Alkoxy mit 1–4 C-Atomen oder Phenyl bedeutet, wobei der Pheylrest durch Fluor, Chlor, Brom, Jod, Cyan, Alkyl mit 1–6 C-Atomen, Alkoxy mit 1–4 C-Atomen, Phenyl oder Phenoxy substituiert sein kann,

$R^2$ die gleichen Bedeutungen wie $R^1$ hat, wobei $R^1$ und $R^2$ im Einzelfall gleich oder verschieden sind,

$R^3$ Wasserstoff, Halogen, Alkyl mit 1–4 C-Atomen, Alkoxy mit 1–4 C-Atomen, Cyan, Phenyl oder Phenoxy bedeutet, wobei der Phenyl- oder der Phenoxyrest selbst wieder durch Halogen oder Alkyl mit 1–4 C-Atomen substituiert sein kann,

n eine ganze Zahl von 1 bis 5,

X S oder O und

Y N oder CH bedeutet.

2. Fungizid, enthaltend eine Azolyl-glykolsulfonat gemäß Anspruch 1.

3. Fungizid, enthaltend einen festen oder flüssigen Trägerstoff und ein Azolyl-glykolsulfonat gemäß Anspruch 1.

4. Verfahren zur Herstellung eines Fungizids, dadurch gekennzeichnet, daß man einen festen oder flüssigen Trägerstoff vermischt mit einem Azolyl-glykolsulfonat gemäß Anspruch 1.

5. Verfahren zur Bekämpfung von Pilzen, dadurch gekennzeichnet, daß man die Pilze oder die vor Pilzbefall zu schützenden Gegenstände behandelt mit einem Azolyl-glykolsulfonat gemäß Anspruch 1.

6. Verfahren zur Herstellung eines Azolyl-glykolsulfonats gemäß Anspruch 1, dadurch gekennzeichnet, daß man ein Alkoholat der allgemeinen Formel

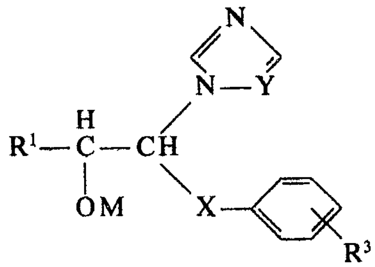

mit einem Sulfonsäurehalogenid der Formel

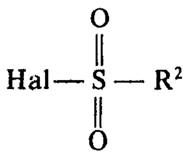

umsetzt, wobei $R^1$, $R^2$, $R^3$, X und Y die im Anspruch 1 angegebenen Bedeutungen haben, Hal ein Halogenatom und M ein Alkali-, ein quartäres Ammonium- oder ein Phosphonium-Ion bedeutet.

**Patentansprüche für den Vertragsstaat: AT**

1. Fungizid enthaltend ein Azolyl-glykolsulfonat der allgemeinen Formel

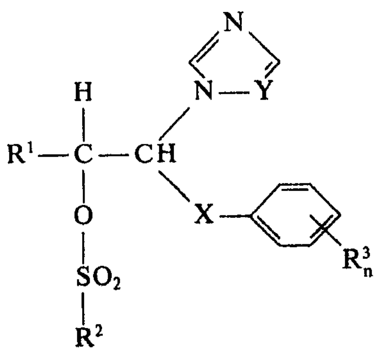

in der

$R^1$ Alkyl mit 1—6 C-Atomen, gegebenenfalls substituiert durch Fluor, Chlor, Brom, Jod oder $R^1$ Alkoxy mit 1—4 C-Atomen oder Phenyl bedeutet, wobei der Phenylrest durch Fluor, Chlor, Brom, Jod, Cyan, Alkyl mit 1—6 C-Atomen, Alkoxy mit 1—4 C-Atomen, Phenyl oder Phenoxy substituiert sein kann,
$R^2$ die gleichen Bedeutungen wie $R^1$ hat, wobei $R^1$ und $R^2$ im Einzelfall gleich oder verschieden sind,
$R^3$ Wasserstoff, Halogen, Alkyl mit 1—4 C-Atomen, Alkoxy mit 1—4 C-Atomen, Cyan, Phenyl oder Phenoxy bedeutet, wobei der Phenyl- oder der Phenoxyrest selbst wieder durch Halogen oder Alkyl mit 1—4 C-Atomen substituiert sein kann,
n eine ganze Zahl von 1 bis 5,
X S oder O und
Y N oder CH bedeutet.

2. Fungizid enthaltend einen festen oder flüssigen Trägerstoff und ein Azolyl-glykolsulfonat gemäß Anspruch 1.

3. Verfahren zur Herstellung eines Fungizids, dadurch gekennzeichnet, daß man einen festen oder flüssigen Trägerstoff vermischt mit einem Azolyl-glykolsulfonat gemäß Anspruch 1.

4. Verfahren zur Bekämpfung von Pilzen, dadurch gekennzeichnet, daß man die Pilze oder die vor Pilzbefall zu schützenden Gegenstände behandelt mit einem Azolyl-glykolsulfonat gemäß Anspruch 1.

5. Verfahren zur Herstellung eines Azolyl-glykolsulfonats gemäß Anspruch 1, dadurch gekennzeichnet, daß daß man ein Alkoholat der allgemeinen Formel

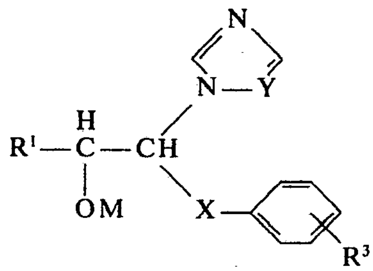

mit einem Sulfonsäurehalogenid der Formel

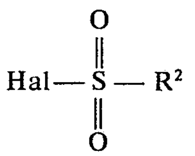

umsetzt, wobei $R^1$, $R^2$, $R^3$, X und Y die im Anspruch 1 angegebenen Bedeutungen haben, Hal ein Halogenatom und M ein Alkali-, ein quartäres Ammonium- oder ein Phosphonium-Ion bedeutet.

**Claims for the Contracting states: BE, CH, DE, FR, GB, IT, LI, LU, NL, SE**

1. An azolylglycol sulfonate of the general formula

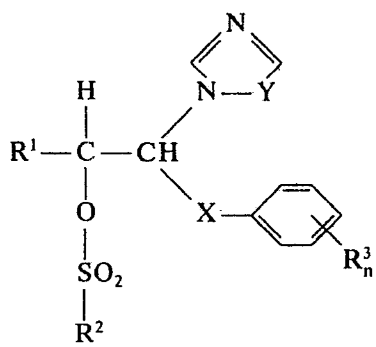

where $R^1$ is alkyl of 1 to 6 carbon atoms, which is unsubstituted or substituted by fluorine, chlorine, bromine or iodine, or is alkoxy of 1 to 4 carbon atoms, or is phenyl which is unsubstituted or substituted by fluorine, chlorine, bromine, iodine, cyano, alkyl of 1 to 6 carbon atoms, alkoxy of 1 to 4 carbon atoms, phenyl or phenoxy, $R^2$ has the same meanings as $R^1$ and $R^1$ and $R^2$ can be identical or different, $R^3$ is hydrogen, halogen, alkyl of 1 to 4 carbon atoms, alkoxy of 1 to 4 carbon atoms, cyano, phenyl or phenoxy, the last two being unsubstituted or substituted by halogen or alkyl of 1 to 4 carbon atoms, n is an integer from 1 to 5, X is S or O and Y is N or CH.

2. A fungicide containing an azolylglycol sulfonate as claimed in claim 1.

3. A fungicide containing a solid or liquid carrier and an azolylglycol sulfonate as claimed in claim 1.

4. A process for producing a fungicide, wherein a solid or liquid carrier is mixed with an azolylglycol sulfonate as claimed in claim 1.

5. A process for combating fungi, wherein the fungi or the objects to be protected against fungus attack are treated with an azolylglycol sulfonate as claimed in claim 1.

6. A process for preparing an azolylglycol sulfonate as claimed in claim 1, wherein an alcoholate of the general formula

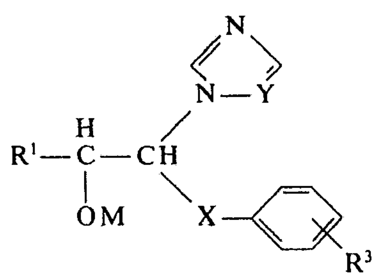

is reacted with a sulfonyl halide of the formula

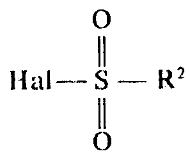

in which formulae $R^1$, $R^2$, $R^3$, X and Y have the meanings given in claim 1, Hal denotes a hologen atom, and M denotes an alkali metal, a quaternary ammonium or a phosphonium ion.

**Claims for the Contracting state: AT**

1. A fungicide containing an azolylglycol sulfonate of the general formula

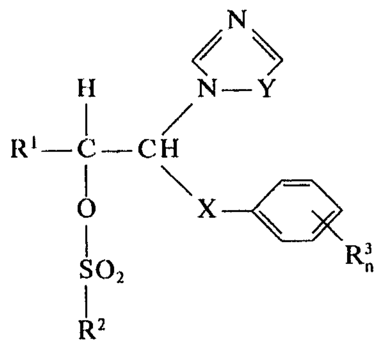

where $R^1$ is alkyl of 1 to 6 carbon atoms, which is unsubstituted or substituted by fluorine, chlorine, bromine or iodine, or is alkoxy of 1 to 4 carbon atoms, or is phenyl which is unsubstituted or substituted by fluorine, chlorine, bromine, iodine, cyano, alkyl of 1 to 6 carbon atoms, alkoxy of 1 to 4 carbon atoms, phenyl or phenoxy, $R^2$ has the same meanings as $R^1$ and $R^1$ and $R^2$ can be identical or different, $R^3$ is hydrogen, halogen, alkyl of 1 to 4 carbon atoms, alkoxy of 1 to 4 carbon atoms, cyano, phenyl or phenoxy, the last two being unsubstituted or substituted by halogen or alkyl of 1 to 4 carbon atoms, n is an integer from 1 to 5, X is S or O and Y is N or CH.

2. A fungicide containing a solid or liquid carrier and an azolylglycol sulfonate as defined in claim 1.

3. A process for producing a fungicide, wherein a solid or liquid carrier is mixed with an azolylglycol sulfonate as defined in claim 1.

4. A process for combating fungi, wherein the fungi or the objects to be protected against fungus attack are treated with an azolylglycol sulfonate as defined in claim 1.

5. A process for preparing an azolylglycol sulfonate as defined in claim 1, wherein an alcoholate of the general formula

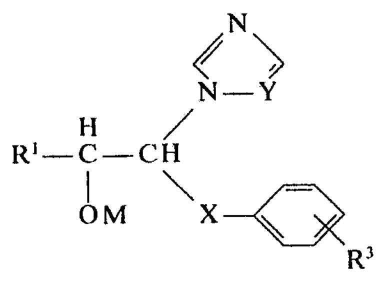

is reacted with a sulfonyl halide of the formula

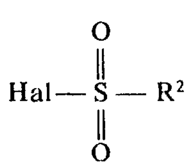

in which formulae $R^1$, $R^2$, $R^3$, X and Y have the meanings given in claim 1, Hal denotes a halogen atom, and M denotes an alkali metal, a quaternary ammonium or a phosphonium ion.

**Revendications pour les Etats contractants: BE, CH, DE, FR, GB, IT, LI, LU, NL, SE**

1. Azolyl-glycolsulfonate de la formule générale suivante

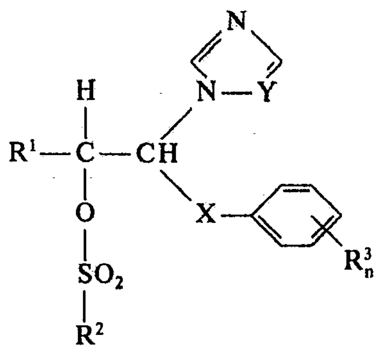

dans laquelle

$R^1$ représente un radical possédant de 1 à 6 atomes de carbone, éventuellement substitué par du fluor, du chlore, du brome, de l'iode, ou bien $R^1$ représente un radical alcoxy possédant de 1 à 4 atomes de carbone ou un radical phényle, où le radical phényle peut être substitué par du fluor, du chlore, du brome, de l'iode, des radicaux cyano, alkyle possédant de 1 à 6 atomes de carbone, alkoxy possédant de 1 à 4 atomes de carbone, phényle ou phénoxy,
$R^2$ possède les mêmes significations que $R^1$, où $R^1$ et $R^2$ peuvent, en l'espèce, être identiques ou différents,
$R^3$ représente un atome d'hydrogène, un atome d'halogène, un radical alkyle possédant de 1 à 4 atomes de carbone, alcoxy possédant de 1 à 4 atomes de carbone, cyano phényle ou phénoxy, où le radical phényle ou le radical phénoxy eux-mêmes peuvent, à leur tour, être substitués par des halogènes ou des radicaux alkyle possédant de 1 à 4 atomes de carbone,
n représente un nombre entier dont la valeur varie de 1 à 5,
X représente un atome de soufre ou d'oxygène et
Y représente un atome d'azote ou le groupe CH.

2. Fongicide contenant un azolyl-glycolsulfonate suivant la revendication 1.

3. Fongicide contenant un support ou véhicule solide ou liquide et un azolyl-glycolsulfonate suivant la revendication 1.

4. Procédé de préparation d'un fongicide, caractérisé en ce que l'on mélange un support ou véhicule solide ou liquide à un azolyl-glycolsulfonate suivant la revendication 1.

5. Procédé de lutte contre les champignons, caractérisé en ce que l'on traite les champignons ou les objects à protéger contre une attaque par des champignons, par un azolyl-glycolsulfonate suivant la revendication 1.

6. Procédé de préparation d'un azolyl-glycolsulfonate suivant la revendication 1, caractérisé en ce que l'on fait réagir un alcoolate de la formule générale:

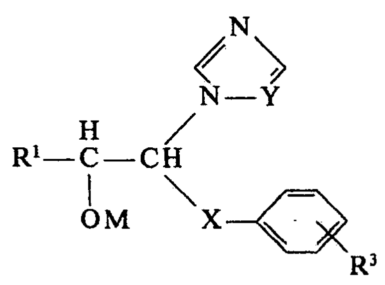

sur un halogénure d'acide sulfonique de la formule

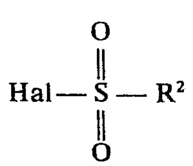

formules dans lesquelles $R^1$, $R^2$, $R^3$, X et Y possèdent des significations qui leur ont été attribuées dans

la revendication 1, Hal représente un atome d'hydrogène et M représente un ion métal alcalin, ammonium quaternaire ou phosphonium.

**Revendications pour l'Etat contractant: AT**

1. Fongicide contenant un azolyl-glycolsulfonate de la formule générale

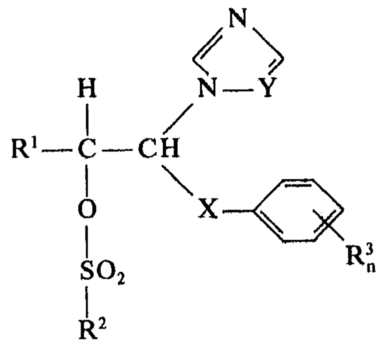

dans laquelle

$R^1$ représente un radical alkyle possédant de 1 à 6 atomes de carbone, éventuellement substitué par du fluor, du chlore, du brome, de l'iode ou bien $R^1$ représente un radical alcoxy possédant de 1 à 4 atomes de carbone ou un radical phényle, où le radical phényle peut être substitué par du fluor, du chlore, du brome, de l'iode, des radicaux cyano, alkyle possédant de 1 à 6 atomes de carbone, alkoxy possédant de 1 à 4 atomes de carbone, phényle ou phénoxy,

$R^2$ possède les mêmes significations que $R^1$, où $R^1$ et $R^2$ peuvent, en l'espèce, être identiques ou différents,

$R^3$ représente un atome d'hydrogène, un atome d'halogène, un radical alkyle possédant de 1 à 4 atomes de carbone, alcoxy possédant de 1 à 4 atomes de carbone, cyano, phényle ou phénoxy, où le radical phényle ou le radical phénoxy eux-mêmes peuvent, à leur tour, être substitués par des halogènes ou des radicaux alkyle possédant de 1 à 4 atomes de carbone,

n représente un nombre entier dont la valeur verie de 1 à 5,

X représente un atome de soufre ou d'oxygène et

Y représente un atome d'azote ou le groupe CH.

2. Fongicide contenant un véhicule ou support solide ou liquide et un azolyl-glycolsulfonate, suivant la revendication 1.

3. Procédé de préparation d'un fongicide, caractérisé en ce que l'on mélange un support ou véhicule solide ou liquide et un azolyl-glycolsulfonate suivant la revendication 1.

4. Procédé de lutte contre les champignons, caractérisé en ce que l'on traite les champignons ou les objets à protéger contre une attaque par des champignons, par un azolyl-glycolsulfonate suivant la revendication 1.

5. Procédé de préparation d'un azolyl-glycolsulfonate suivant la revendication 1, caractérisé en ce que l'on fait réagir un alcoolate de la formule générale:

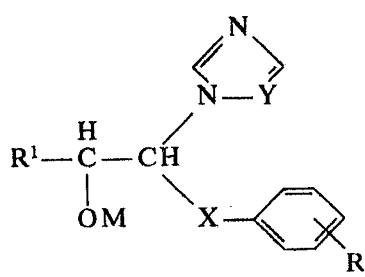

sur un halogénure d'acide sulfonique de la formule

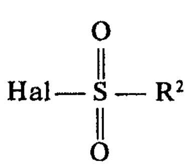

formules dans lesquelles $R^1$, $R^2$, $R^3$, X et Y possèdent des significations qui leur ont été attribuées dans la revendication 1, Hal représente un atome d'hydrogène et M représente un ion métal alcalin, ammonium quaternaire ou phosphonium.